# EUROPÄISCHE PATENTSCHRIFT

(11) **EP 3 533 394 B1**
(45) Veröffentlichungstag und Bekanntmachung des Hinweises auf die Patenterteilung: **22.01.2025**
(21) Anmeldenummer: 19156847.6
(22) Anmeldetag: 13.02.2019
(51) Int. Cl.: A61B 5/087, A61B 5/091, A61B 5/00, G16H 10/00, A61M 16/00, A61B 5/08

(54) **ATEMGASANALYSATOR ZUR AUSWERTUNG EINES ATEMGASES**
RESPIRATORY GAS ANALYSER FOR EVALUATING A RESPIRATORY GAS
ANALYSEUR DE GAZ RESPIRATOIRE PERMETTANT D'ÉVALUER UN GAZ RESPIRATOIRE

(30) Priorität: 28.02.2018 DE 102018001557
(43) Veröffentlichungstag der Anmeldung: 04.09.2019
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Löwenstein Medical IP

(56) Entgegenhaltungen:
- EP-A1- 2 465 434
- EP-A1- 2 818 111
- WO-A1-2011/006199
- WO-A1-2017/132726
- WO-A1-2018/011801
- US-A1- 2014 088 373

## Beschreibung

Die vorliegende Erfindung betrifft einen Atemgasanalysator zur Auswertung eines Atemgases.

Eine Vielzahl an Menschen leiden unter einem schlechten Schlaf, der zu nachlassender Konzentration und verminderter geistiger Leistungsfähigkeit führt. Ein schlechter Schlaf kann ebenso für Störungen anderer Funktionen wie beispielsweise Muskelspannung, Atmung, Herzschlag, Blutdruck, Körpertemperatur, Hormone und Stoffwechsel verantwortlich sein. Die Ursachen für einen schlechten Schlaf können dabei vielfältig sein. Oftmals resultiert ein schlechter Schlaf aus einer Schlafstörung des Patienten. Ein schlechter Schlaf kann jedoch auch vorliegen, obwohl keine Schlafstörung ermittelt werden konnte.

Eine mögliche Ursache für eine Schlafstörung kann eine Unregelmäßigkeit in der Atmung (Atemstörungen) sein. Die häufigste Schlafstörung ist die obstruktive Schlaf-Apnoe (Obstructive Sleep Apnea, OSA); dabei ist der Atemfluss vorübergehend entweder wesentlich vermindert (Hypopnoe) oder vollständig unterbrochen (Apnoe). Der infolgedessen auftretende Sauerstoffmangel erzeugt eine Stresssituation, die einen erholsamen Schlaf unterbindet und das Herz mittelfristig schädigt.

Um eine Aussage über das Ausmaß einer Schlafstörung eines Patienten treffen zu können, um diese entsprechend therapieren bzw. beheben zu können, ist es notwendig, eine Schlafdiagnostik durchzuführen.

Eine Schlafdiagnostik kann beispielsweise durch Polygraphiegeräte oder Beatmungsgeräte erfolgen.

Die Polygraphiegeräte sind zur Durchführung einer Polygraphie eingerichtet. Bei der Polygraphie handelt es sich um ein Verfahren, welches vornehmlich in der Heimdiagnostik Anwendung findet. Dabei wird das für die Polygraphie eingerichtete Polygraphiegerät / Messgerät dem Patienten mit nach Hause gegeben. Das Polygraphiegerät / Messgerät zeichnet Informationen über Parameter wie beispielsweise eine nächtliche Sauerstoffsättigung, eine Pulsfrequenz, eine Lage des Körpers des Patienten während des Schlafes sowie über eine Atmung des Patienten im Schlaf inklusive Schnarchen auf. Je nach Art des Polygraphiegerätes / Messgerätes kann das Polygraphiegerät / Messgerät ein Elektrokardiogramm (EKG) oder Elektromyogramm (EMG) umfassen, welches eine nächtliche Herzaktivität bzw. eine Beinmuskulaturaktivität des Patienten im Schlaf erfassen kann. Die erfassten Parameter können für eine Aussage über das Ausmaß der Schlafstörung verwendet werden. Ferner kann über die erfassten Parameter die Funktion der Beatmungsgeräte bzw. der Beatmungstherapie kontrolliert werden.

Polygraphiegeräte werden oftmals zusammen mit Beatmungsgeräten verwendet. Die in der Polygraphie genutzten Beatmungsgeräte sind dabei zur Durchführung der Standardtherapien, insbesondere der CPAP-Therapie (engl. Continuous Positive Airway Pressure), der APAP-Therapie (engl. Automatic Positive Airway Pressure) und der Bi-Level-Therapie eingerichtet. Einige der beschriebenen Beatmungsgeräte bzw. -therapien, insbesondere der APAP-Therapie und / oder der Bilevel-Therapie, können ebenfalls eingerichtet sein, Atemgasparameter zu erfassen, um beispielsweise einen benötigten Atemgasdruck eines Patienten von Atemzug zu Atemzug zu ermitteln und dem Patienten entsprechend zuzuführen. Hierfür umfassen die Beatmungsgeräte in der Regel mindestens einen Drucksensor und / oder einen Flusssensor zur Ermittlung eines Atemgasdrucks bzw. eines Atemgasvolumens.

Die bisher bekannten Polygraphie- und Beatmungsgeräte können somit eingerichtet sein, Atemgasparameter zu erfassen und ein Ausmaß einer Schlafstörung, die durch schlafbezogene Atemstörungen hervorgerufen wird, zu ermitteln. Sie können jedoch keine Schlafstörungen ermitteln, die nicht atemstörungsbedingt sind. Somit können die bisher bekannten Polygraphie- und Beatmungsgeräte nicht auf eine Schlafqualität (schlechter oder guter Schlaf) des Patienten schließen.

Um bei der Schlafdiagnostik neben dem Ausmaß der Schlafstörung ebenfalls eine Aussage über die Schlafqualität des Patienten treffen zu können, ist es derzeit notwendig eine Polysomnographie in einem Schlaflabor durchzuführen. Bei der Polysomnographie werden Informationen / Parameter über den aktuellen Schlafzustand sowie über die Schlafqualität des Patienten kontinuierlich mittels eines aufwendigen Messaufbaus in einem Schlaflabor erfasst.

Die schlafmedizinisch überwachte Polysomnographie gilt bisher als Grundinstrument und als Referenzmethode bei der apparativen Diagnostik von Schlafstörungen. Die Polysomnographie umfasst dabei u.a. die Aufzeichnungen von Schlaf-EEG (Hirnstrombild), EOG (Augenbewegung), EMG (Muskelspannung), EKG (Herzrhythmus), Atemfluss, Atmungsanstrengung, Sauerstoffsättigung, Körpertemperatur, Körperlage und Beinbewegung. Vor der Durchführung einer Polysomnographie im Schlaflabor wird der Patient in der Regel für einen Tag beobachtet, um seine Aktivitäten und Gewohnheiten zu erkennen. Normalerweise verbringt der Patient anschließend zwei Nächte im Schlaflabor.

Die Erfassung der Schlafqualität mittels der Polysomnographie hat den Nachteil, dass das Polysomnographiegerät durch den aufwendigen Aufbau sehr kostenintensiv ist. Das Verfahren der Polysomnographie ist sehr zeitaufwendig, da der Patient mehrtägig in dem Schlaflabor stationär aufgenommen werden muss. Eine Heimanwendung durch den Patienten ist somit nicht möglich. Zudem kann eine Auswertung der erhobenen Informationen / Parameter erst am nächsten Tag erfolgen.

Die WO 2011/006199 A1 offenbart eine Ermittlung der Schlafqualität basierend auf dem Flusssignal. Dazu wird der Atemgasfluss sehr detailliert analysiert, um definierte Atemereignisse aus dem Flusssignal zu erkennen und um daraus auf Schlafstadien zu schließen.

Die EP 2 465 434 A1 offenbart eine Konfiguration, die in der Lage ist, einen Erkennungstest für ein Komfortniveau einschließlich der Schlafqualität durchzuführen, das zu Hause messbar ist, ohne dass die Messung von Gehirnwellen oder ein Elektrokardiogramm erforderlich ist.

Es ist daher Aufgabe der vorliegenden Erfindung, eine Vorrichtung bereitzustellen, welche eingerichtet ist, einem Patienten bzw. betreuendem Fachpersonal neben einer Aussage über ein Ausmaß einer Schlafstörung (schlafbezogene Atemstörung) des Patienten auch eine vereinfachte und unmittelbare Aussage über seine Schlafqualität bereitzustellen.

Gegenstand der vorliegenden Erfindung ist ein Atemgasanalysator zur Auswertung eines Atemgases nach Anspruch **1**. Weitere Ausführungsformen werden durch abhängige Ansprüche **2-9** definiert.

Gegenstand der Erfindung ist auch ein Verfahren zur Steuerung eines Atemgasanalysators nach Anspruch **10.** Weitere Ausführungsformen werden definiert in Ansprüchen **11-12.**

Im Folgenden werden anhand von stark vereinfachten schematischen Darstellungen bevorzugte Ausführungsbeispiele der Erfindung näher erläutert. Es zeigt:
- Fig. 1: einen grundsätzlichen Aufbau einer Vorrichtung zur Beatmung,
- Fig. 2: einen beispielhaften schematischen Aufbau eines erfindungsgemäßen Atemgasanalysators zur Bestimmung einer Schlafqualität,
- Fig. 3: einen beispielhaften Aufbau eines erfindungsgemäßen Atemgasanalysators,
- Fig. 4: eine Ausführungsform einer Ermittlung eines erfindungsgemäßen Schlafqualitätsindexes.

In den Figuren weisen dieselben konstruktiven Elemente jeweils dieselben Bezugsziffern auf.

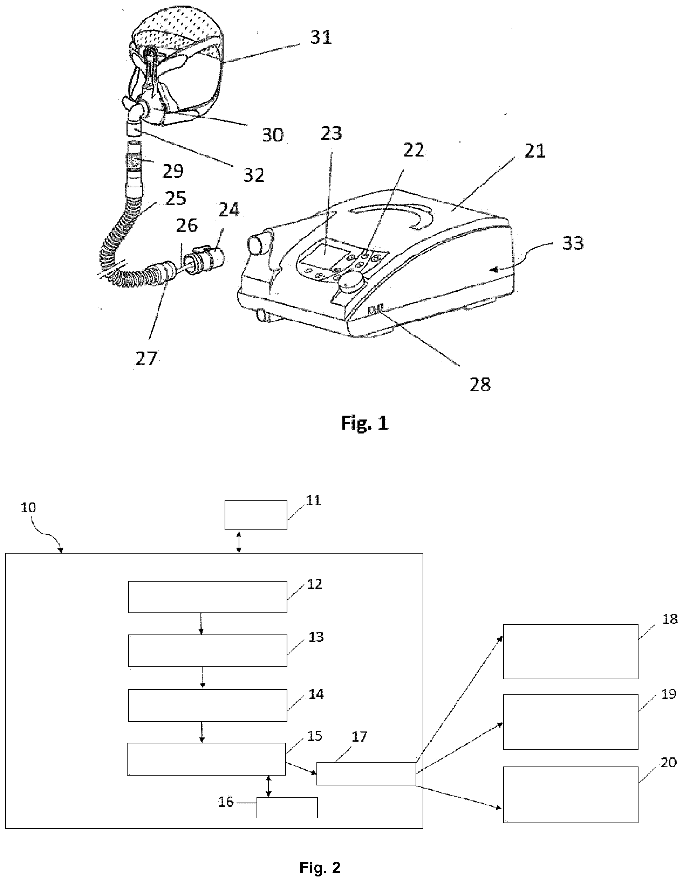

Figur 1 zeigt den grundsätzlichen Aufbau einer Vorrichtung zur Beatmung. Im Bereich eines Gerätegehäuses 21 des Beatmungsgerätes 33, mit einer Atemgasquelle im Geräteinnenraum, sind ein Bedienelement 22 und ein Bedien-und Informationssystem 23 bestehend aus einer Anzeige, welche eine, beispielsweise berührungsempfindliche, Eingabeeinheit mit zumindest einem Bedienfeld aufweisen kann. Über eine Kopplung 24 wird ein Verbindungsschlauch 25 angeschlossen. Entlang des Verbindungsschlauches 25 kann ein zusätzlicher Druckmessschlauch 26 verlaufen, der über einen Druckeingangsstutzen 27 mit dem Gerätegehäuse 21 verbindbar ist. Zur Ermöglichung einer Datenübertragung weist das Gerätegehäuse 21 zumindest eine oder auch eine Vielzahl von Schnittstelle(n) 28 auf.

Ein Anfeuchter kann zudem adaptiert werden. Im Bereich einer dem Gerätegehäuse 21 abgewandten Ausdehnung des Verbindungsschlauches 25 ist ein Ausatmungselement 29 angeordnet. Ebenfalls kann ein Ausatemventil verwendet werden.

Figur 1 zeigt darüber hinaus ein als Beatmungsmaske 30 ausgebildetes PatientenInterface, das als Nasalmaske realisiert ist. Eine Fixierung im Bereich eines Kopfes eines Patienten kann über eine Kopfhaube 31 erfolgen. Im Bereich seiner dem Verbindungsschlauch 25 zugewandten Ausdehnung weist das Patienten-Interface 30 ein Kupplungselement 32 auf.

Über die Schnittstelle 28 kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein.

Bevorzugt ist auch ein Kartenschacht vorgesehen. Die Schnittstelle 28 kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern. Statt einer Schnittstelle 28 kann auch eine Vielzahl von Schnittstellen vorgesehen sein.

Über die Schnittstelle 28 - beispielsweise als Kartenschacht oder USB ausgeführt - können auch therapiefremde Daten in das erfindungsgemäße Beatmungsgerät geladen werden beziehungsweise von diesem ausgeführt werden. Der Anwender muss - werden vom Gerät externe Speichermedien erkannt - eine Abfrage im Bedienfeld bestätigen, woraufhin die Daten wahlweise im Bereich des Beatmungsgerätes gespeichert oder ausgeführt werden.

Über die Schnittstelle 28 kann die Eingabe und / oder Ausgabe von telemedizinischen Daten erfolgen. Dazu werden über die Schnittstelle beispielsweise Mobilfunk- oder Nahfeldfunkdaten empfangen / gesendet oder WLAN oder Bluetooth oder Netzwerkdaten.

Das Beatmungsgerät 33 ist so ausgelegt, dass es über einen Schlauch und ein Patienteninterface 30 mit einem Patienten verbunden werden kann, um eine Beatmung bereitzustellen. Es umfasst eine Quelle für Atemgas, die beispielsweise als ein Elektromotor mit Gebläserad ausgebildet ist, und eine Einrichtung zur Ermittlung von Druck und / oder Fluss und / oder Volumen des Atemgases, sowie eine Steuereinheit, die so ausgelegt ist, dass sie für jeden Atemzyklus auf der Basis eines vorbestimmten Wertes für den Patienten und / oder auf Basis von Messsignalen für die Parameter Druck und / oder Fluss und / oder Volumen einen Atemgasdruck bestimmt und die Quelle für Atemgas derart reguliert, dass der Atemgasdruck erzeugt wird.

Figur 2 zeigt einen beispielhaften schematischen Aufbau des erfindungsgemäßen Atemgasanalysators 10, der eine Vorverarbeitungseinheit 12, einen Zwischenspeicher 13, eine Integratoreinheit 14 und eine Detektoreinheit 15 umfasst.

Der Atemgasanalysator 10 ist in einem - in Fig. 1 gezeigten - Beatmungsgerät angeordnet. Der Atemgasanalysator 10 kann aus dem Atemgas mindestens ein Schlafstadium ermitteln und basierend auf dem Schlafstadium eine Schlafqualität ermitteln.

Der Atemgasanalysator 10 ist eingerichtet, den mindestens einen Atemgasparameter von dem Messsystem 11 zu beziehen. Beispielsweise ist ein Atemgasparameter ein Atemgasvolumen. Das Messsystem 11 ist extern des Atemgasanalysators 10 angeordnet. Beispielsweise kann das Messsystem 11 in einem PAP- / Beatmungsgerät, einem Diagnose- / Polygraphiegerät, einem Polysomnographiegerät oder an einem Patienten angeordnet sein.

In dem vorliegenden Ausführungsbeispiel ist die Vorverarbeitungseinheit 12 eingerichtet, basierend auf dem bezogenen Atemgasvolumen mindestens einen normierten Wert des Atemgasvolumens, beispielsweise über einen Zeitraum von 1 Minute, zu ermitteln.

Die Zwischenspeichereinheit 13 ist eingerichtet, den normierten Wert zwischenzuspeichern und der Detektoreinheit 14 zur Verfügung zu stellen. Die Detektoreinheit 14 bezieht aus dem Zwischenspeicher den mindestens einen normierten Wert und ermittelt basierend auf diesem mindestens ein Stabilitätslevel. Das Stabilitätslevel kann beispielsweise durch einen Vergleich eines aktuellen Wertes, beispielsweise des Atemgasvolumens pro Minute, mit einem entsprechenden normalisierten Wert des Atemgasvolumens, ermittelt werden. Vorzugsweise ist die Detektoreinheit eingerichtet, das ermittelte Stabilitätslevel an die Zwischenspeichereinheit zur Zwischenspeicherung zurück zu senden. Basierend auf dem mindestens einen ermittelten Stabilitätslevel ist die Detektoreinheit eingerichtet, eine Einteilung in mindestens ein Schlafstadium vorzunehmen. Beispielsweise kann die Detektoreinheit eingerichtet sein, bei einem Stabilitätslevel über einem vorgegebenen Schwellenwert zwischen 0 und 1, beispielsweise bei 0,8 auf einen Tiefschlaf zu schließen.

Die Integratoreinheit 15 ist eingerichtet, basierend auf dem mindestens einen, durch die Detektoreinheit 14 ermittelten, Schlafstadium, eine Schlafqualität zu ermitteln. Die Schlafqualität wird als Schlafqualitätsindex wiedergegeben. Der Schlafqualitätsindex kann auf dem - in Fig. 1 gezeigten - Bedien-und Informationssystem / Anzeige 23 oder auf dem Endgerät 20 mit einer Skala zwischen 0 und 100 angegeben oder als Grafik dargestellt werden. Die Skala ist anpassbar. Typischerweise deutet ein niedrigerer Schlafqualitätsindex auf einen schlechten Schlaf hin, während ein höherer Schlafqualitätsindex auf einen besseren Schlaf hindeutet. Der Schlafqualitätsindex kann als Zahlenwert und / oder als Grafik dem Patienten und / oder einem Fachpersonal zur Verfügung gestellt werden.

Der Atemgasanalysator 10 kann eingerichtet sein, den ermittelten Schlafqualitätsindex und / oder mindestens ein ermitteltes Schlafstadium an eine Bewertungseinheit 16 zu übermitteln, um diese mit Referenzwerten, beispielsweise für ein Alter, zu vergleichen und zu bewerten. Die Bewertereinheit 16 kann intern oder extern des Atemgasanalysator ausgebildet sein. Die Bewertungseinheit 16 übermittelt, das mindestens eine bewertete Schlafstadium und / oder den bewerteten Schlafqualitätsindex an die Integratoreinheit 15 zurück. Der Atemgasanalysator 10 ist eingerichtet, das mindestens eine bewerte Schlafstadium oder den bewerteten Schlafqualitätsindex an das - in Fig. 1 gezeigte - Bedien- und Informationssystem / Anzeige 23 oder mittels einer Schnittstelle 17 an das Endgerät 20 zu übermitteln.

Die Schnittstelle 17 ist somit eingerichtet, mit einem Endgerät 20 zu interagieren, um die Schlafqualität auf dem Endgerät 20 darzustellen. Die Schnittstelle 17 umfasst dabei ein Modem, wodurch der Atemgasanalysator 10 mit mindestens einem Endgerät 20 in einem Netzwerk kommunizieren kann. Die Schnittstelle 17 ist in der Regel über das Bedien- und Informationssystem / Anzeige 23 ansteuerbar. Die Schnittstelle 17 kann der - in Figur 1 gezeigten Schnittstelle 28 entsprechen. Basierend auf dem ermittelten Schlafstadium und / oder der ermittelten Schlafqualität und / oder dem ermittelten Stabilitätslevel ist der Atemgasanalysator 10 eingerichtet, ein Polygraphiegerät / Diagnosegerät und/oder ein PAP- / Beatmungsgerät zu steuern.

Der in Figur 2 gezeigte Atemgasanalysator 10 kann in einem gemäß Figur 1 beschriebenem Beatmungsgerät 13 verwendet werden. Das Beatmungsgerät 13 ist zur Verwendung mit einer über einen Beatmungsschlauch verbundenen Beatmungsmaske 10 eingerichtet. Die Beatmungsmaske 10 und der Beatmungsschlauch 5 sind dabei individuell wählbar. Das Beatmungsgerät 13 kann ein Beatmungsgerät der CPAP-Klasse, der APAP-Klasse oder der Bilevel-Klasse sein. Optional kann der erfindungsgemäße Atemgasanalysator 10 für eine mindestens drei Beatmungsdrucklevel umfassende Therapie eingesetzt werden. Der erfindungsgemäße Atemgasanalysator 10 ist ebenso zur Verwendung in einem Diagnose- / Polygraphiegerät geeignet.

Ferner zeigt Figur 2 einen beispielhaften Ablauf des erfindungsgemäßen Verfahrens zur Steuerung eines Atemgasanalysators.

In einem ersten Schritt des Verfahrens wird mindestens ein Atemgasparameter durch den Atemgasanalysator 10 von dem Messsystem 11 bezogen. Beispielsweise bezieht der Atemgasanalysator 10 einen durch eine Atemgasflusssensor des Messsystems 11 ermittelten Atemgasparameter, beispielsweise ein Atemgasvolumen pro Minute.

In einem weiteren Schritt wird der mindestens eine Atemgasparameter durch die Vorverarbeitungseinheit 12 zu mindestens einer Kennzahl oder zu mindestens einem Atemgassignal vorverarbeitet. Ein Atemgasparameter ist beispielsweise ein Volumen des aktuellen Atemzuges oder einer aktuelle Atemfrequenz sein. In der Regel ermittelt die Vorverarbeitungseinheit 12 beispielsweise aus dem Atemgasvolumen einen normierten Wert über einen vorbestimmten Zeitraum, beispielsweise eine Minute.

In einem weiteren Schritt wird in der Regel zumindest der normierte Wert, vorzugsweise jedoch auch die mindestens eine Kennzahl oder das mindestens eine Atemgassignal in der Zwischenspeichereinheit 13 zwischengespeichert. In einem weiteren Schritt werden die mindestens eine Kennzahl und / oder das mindestens eine Atemgassignal der Detektoreinheit 14, beispielsweise über einen Zeitraum von 5 Sekunden bis 5 Minuten, der Detektoreinheit 14 zur Verfügung gestellt.

In der Regel bezieht die Detektoreinheit 14 aus dem Zwischenspeicher den mindestens einen normierten Wert und ermittelt, basierend auf diesem, mindestens ein Stabilitätslevel. Das Stabilitätslevel wird beispielsweise durch einen Vergleich eines aktuellen Wertes, beispielsweise des Atemgasvolumens pro Minute, mit einem entsprechenden normalisierten Wert des Atemgasvolumens, ermittelt. In der Regel sendet die Detektoreinheit das ermittelte Stabilitätslevel an die Zwischenspeichereinheit zur Zwischenspeicherung zurück. Basierend auf dem mindestens einen ermittelten Stabilitätslevel nimmt die Detektoreinheit eine Einteilung in mindestens ein Schlafstadium vor.

In einem weiteren Schritt wird durch die Integratoreinheit 15 aus dem mindestens einen ermittelten Schlafstadium eine Schlafqualität bestimmt. Dazu werden die zu jedem Zeitpunkt von der Detektoreinheit ermittelten Stabilitätslevel oder Schlafstadien oder Zugehörigkeiten zu Schlafstadien über die gesamte Nacht aufsummiert.

In einem weiteren Schritt wird die mindestens eine ermittelte Schlafqualität als Schlafqualitätsindex dargestellt. Der Schlafqualitätsindex kann aus einem Integral des Kurvenverlaufs der aufsummierten Stabilitätslevel ermittelt werden. Der Schlafqualitätsindex kann dabei als Zahlenwert und / oder als Grafik dargestellt werden. Typischerweise deutet ein niedrigerer Schlafqualitätsindex auf einen schlechten Schlaf hin, während ein höherer Schlafqualitätsindex auf einen besseren Schlaf hindeutet.

In einem weiteren Schritt kann der Atemgasanalysator den ermittelten Schlafqualitätsindex und / oder das mindestens eine ermittelte Schlafstadium und / oder das mindestens eine Stabilitätslevel an eine Bewertereinheit 16 zur Bewertung übersenden. Die Bewertereinheit 16 analysiert den ermittelten Schlafqualitätsindex und / oder das mindestens eine ermittelte Schlafstadium und / oder das mindestens eine Stabilitätslevel und vergleicht diese beispielsweise mit alterstypischen hinterlegten Werten. Optional kann die Bewertereinheit 16 den ermittelten Schlafqualitätsindex oder das mindestens eine ermittelte Schlafstadium mit alternativen Werten vergleichen. Typischerweise übermittelt die Bewertereinheit 16 die bewertete Schlafqualität und / oder das mindestens eine bewertete Schlafstadium und / oder das mindestens eine Stabilitätslevel an den Atemgasanalysator 10 zurück. In einer Weiterbildung des erfindungsgemäßen Verfahrens wird durch den Atemgasanalysator 10 aufgrund des mindestens einen ermittelten Schlafstadiums und / oder der ermittelten Schlafqualität ein Beatmungsgerät und / oder ein Polygraphiegerät 18, 19 und / oder ein Polysomnographiegerät gesteuert. Der ermittelte Schlafqualitätsindex und / oder das mindestens eine Schlafstadium können beispielsweise eine Rückmeldung über die Einstellung des - in Fig. 1 gezeigten - Beatmungsgerätes 33 geben. Basierend auf dem ermittelten Schlafstadium und / oder dem ermittelten Schlafqualitätsindex kann der Atemgasanalysator 10 die Einstellungen des - in Fig. 1 gezeigten - Beatmungsgerätes 33 anpassen.

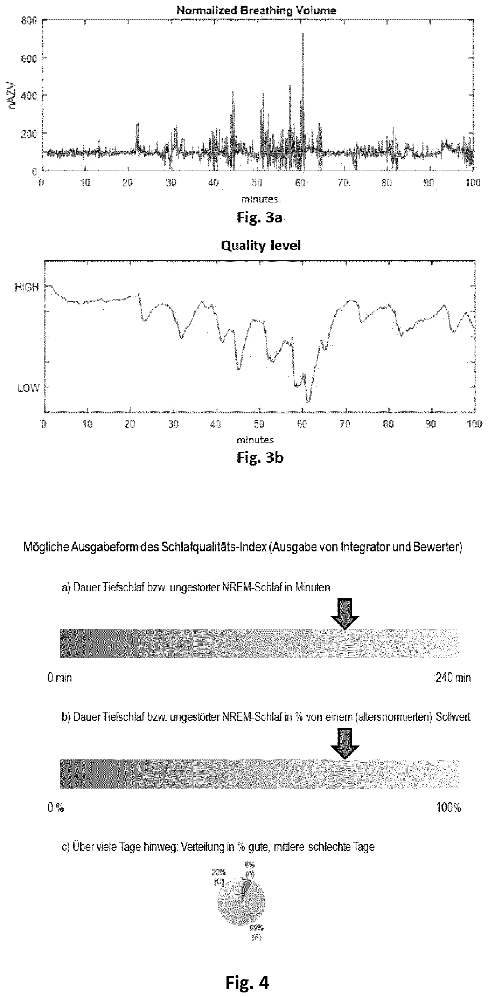

Die Figur 3a zeigt ein beispielhaft ermitteltes normalisiertes Atemgasvolumen (Normalized Breathing Volume). Dabei ist auf der Y-Achse eine Prozentangabe des normalisierten Atemgasvolumens und auf der X-Achse eine Zeitspanne zwischen 0 Sekunden und 100 Minuten aufgetragen. Das in Figur 3a gezeigte normalisierte Atemgasvolumen wird durch eine - in Figur 2 gezeigte - Vorverarbeitungseinheit 12 mit den voranstehend beschriebenen Merkmalen ermittelt.

Die Figur 3b zeigt einen beispielhaften Verlauf eines ermittelten Stabilitätslevels. Dabei ist auf der Y-Achse das Stabilitätslevel mit der Einteilung hoch (high) und niedrig (Low) aufgetragen und auf der X-Achse ist eine Zeitspanne zwischen 0 und 100 Minuten aufgetragen. Das in Fig. 3b gezeigte Stabilitätslevel wird durch eine - in Figur 2 gezeigte - Integratoreinheit 15 mit den voranstehend beschriebenen Merkmalen ermittelt. Bei einem stabilen Atemgasvolumen ist das Schlafqualitätslevel / Qualitäts-Level eher hoch, bei einem schwankenden Atemgasvolumen eher niedrig.

Figur 4 zeigt eine beispielhafte Darstellung des erfindungsgemäßen Schlafqualitätsindex. Dargestellt sind eine Skala a) und eine Skala b) sowie ein Tortendiagramm c). Die Skala a) ist in einen Zeitraum von 0 bis 240 Minuten unterteilt. Skala a) zeigt eine Dauer einer Tiefschlafphase bzw. eines ungestörten NREM-Schlafes in Minuten. Skala b) ist korrespondierend zu Skala a) angeordnet und ist prozentual eingeteilt von 0% bis 100%. Skala b) zeigt die Dauer der Tiefschlafphase bzw. des ungestörten NREM Schlafes in Prozent von einem altersnormierten Sollwert. Das Tortendiagramm c) zeigt eine Darstellung von Verteilungen über einen bestimmten Zeitraum in Prozent. Das Tortendiagramm ist beispielsweise in gute, mittlere und schlechte Tage eingeteilt. Die in Figur 4 dargestellte Verteilung / Ausgabe des erfindungsgemäßen Schlafindexes ist beispielhaft. Die Ausgabe bzw. Darstellung des Schlafqualitätsindex ist anpassbar.

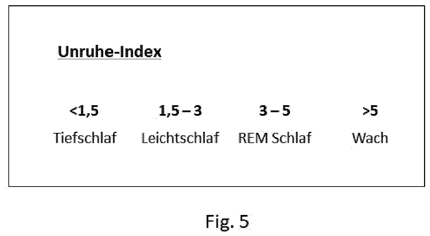

Figur 5 zeigt eine bevorzugte Einteilung des Stabilitätslevels in Schlafstadien zur Bestimmung des erfindungsgemäßen Schlafqualitätsindex.

In einem ersten Schritt des Verfahrens wird - wie in Fig. 2 dargestellt - durch den Atemgasanalysator 10 pro Atemzug eines Patienten ein Atemminutenvolumen in Liter/Minute durch eine Messeinheit oder das Messsystem 11 erfasst und berechnet. Dabei wir das eingeatmete Atemvolumen in Litern und die Dauer des Atemzuges in Sekunden erfasst und mit 60 multipliziert. Dadurch wird ein aktuelles Atemminutenvolumen ermittelt.

In einem weiteren Schritt wird zudem ein mittleres Atemminutenvolumen berechnet und laufend aktualisiert. Beispielweise wird immer das mittlere Atemminutenvolumen der letzten 5 Minuten erfasst. Somit wird zusätzlich zu dem aktuellen Atemminutenvolumen durch den Atemgasanalysator ein mittleres Atemminutenvolumen ermittelt.

In einem weiteren Schritt wird das ermittelte aktuelle Atemminutenvolumen normiert in eine Prozentangabe des Mittelwertes. Somit ist das normierte/normalisierte Atemminutenvolumen das Atemminutenvolumen geteilt durch das mittlere Atemminutenvolumen multipliziert mit 100. Beispielsweise kann ein mittleres Atemminutenvolumen 10 l/min sein und ein aktuelles Atemminutenvolumen 11 l/min. Das normierte Atemminutenvolumen liegt bei diesem Beispiel bei 110%.

In einem weiteren Schritt wird eine Auslenkung des normierten Atemminutenvolumens berechnet. Gemäß dem vorgenannten Beispiel beträgt eine Auslenkung eines normierten Atemminutenvolumens von 110%, 10. Wenn das aktuelle Atemminutenvolumen dem mittleren Atemminutenvolumen entspricht, liegt eine Auslenkung von 0 vor.

In einem weiteren Schritt wird die Auslenkung stark tiefpass-gefiltert, z. B. über 2 Minuten mit einem Glättungsfilter zweiter Ordnung. Dies reduziert die Relevanz einzelner großer Auslenkungen. Der geglättete Wert zeigt an, wie weit die Auslenkung im Mittel während der letzten 2 Minuten von 0 entfernt war.Dies wird als Atem-Unruhe-Index wiedergegeben und entspricht einer Tiefpass-Glättung (Auslenkung). Der Unruhe-Index ist ein Kehrwert des Stabilitätslevels. Je nach Ausführungsform wird ein Unruhe-Index (=hoher Wert wach, niedriger Wert Tiefschlaf) oder ein Stabilitätslevel (umgekehrt) als Zwischenergebnis verwendet.

In einem weiteren Schritt wird entsprechend dem ermittelten Wert des Unruhe-Index (der sich nur langsam ändern kann) oder das durch den Unruhe-Index bekannten Stabilitätslevel, wird der aktuelle Atemzug einem Schlafstadium zugeordnet. In der Fig. 5 ist die Einteilung des Unruhe-Index in die jeweiligen Schlafstadien dargestellt. Der Unruhe-Index ist als Zahlwert angegeben.

Am Ende einer Messperiode, beispielsweise einer Nacht, wird die Zeit und der Anteil der Nacht in der Integratoreinheit addiert, in der der Unruhe-Index im Bereich für Tiefschlaf, im Bereich für Leichtschlaf, für REM Schlaf oder dem Wachzustand usw. lag.

In einem weiteren Schritt wird die Schlafqualität in der Regel durch die Dauer des erfassten Tiefschlafs oder dem Verhältnis aus der Dauer des Tiefschlafs und des Altersdurchschnitts bestimmt.

### Bezugszeichenliste

- 10: Atemgasanalysator
- 11: Messsystem
- 12: Vorverarbeitungseinheit
- 13: Zwischenspeichereinheit
- 14: Detektoreinheit
- 15: Integratoreinheit
- 16: Bewertereinheit
- 17: Schnittstelle
- 18: Diagnose- / Polygraphiegerät
- 19: PAP- /Beatmungsgerät
- 20: Endgerät
- 21: Gerätegehäuse
- 22: Bedienelement
- 23: Bedien-und Informationssystem/ Anzeige
- 24: Kopplung
- 25: Verbindungsschlauch
- 26: Druckmessschlauch
- 27: Druckeingangsstutzen
- 28: Schnittstelle(n)
- 29: Ausatmungselement
- 30: Beatmungsmaske
- 31: Kopfhaube
- 32: Kupplungselement
- 33: Beatmungsgerät

## Patentansprüche

1. Atemgasanalysator (10) zur Auswertung eines Atemgases, wobei der Atemgasanalysator (10) dazu eingerichtet ist, mindestens einen Atemgasparameter des Atemgases von einem Messsystem (11) zu beziehen, oder mindestens eine Messeinheit umfasst, die dazu eingerichtet ist, mindestens einen Atemgasparameter aus dem Atemgas zu ermitteln, wobei der Atemgasanalysator (10) umfasst:
- mindestens eine Vorverarbeitungseinheit (12), die dazu eingerichtet ist, den mindestens einen ermittelten oder bereitgestellten Atemgasparameter auszuwerten und mindestens ein Atemgassignal zu ermitteln, wobei das mindestens eine Atemgassignal ein Atemgasvolumen eines aktuellen Atemzuges ist und die Vorverarbeitungseinheit dazu eingerichtet ist, ein aktuelles Atemgasvolumen pro Atemzug als Prozentwert eines Normalwertes eines inspiratorischen Atemminutenvolumens einer bestimmten Zeit zu normieren, um einen normierten Wert, insbesondere ein normiertes Atemgasvolumen, zu ermitteln;
- mindestens eine Zwischenspeichereinheit (13), die dazu eingerichtet ist, den normierten Wert zwischenzuspeichern;
- mindestens eine Detektoreinheit (14), die dazu eingerichtet ist, aus mindestens einem zwischengespeicherten normierten Wert mindestens ein Schlafstadium zu ermitteln, wobei die Detektoreinheit dazu eingerichtet ist, eine Auslenkung des normierten Werts vom Normalwert zu ermitteln, die ermittelte Auslenkung mit einem Tiefpass zu filtern, den derart geglätteten Wert als einen der Auslenkung entsprechenden Atem-Unruhe-Index wiederzugeben und einen aktuellen Atemzug entsprechend dem ermittelten Wert des Atem-Unruhe-Index oder entsprechend einem Stabilitätslevel als Kehrwert des Atem-Unruhe-Index einem Schlafstadium zuzuordnen;
- mindestens eine Integratoreinheit (15), die dazu eingerichtet ist, basierend auf dem mindestens einen durch die Detektoreinheit (14) ermittelten Schlafstadium eine Schlafqualität zu ermitteln, wobei die Integratoreinheit dazu eingerichtet ist, am Ende einer Messperiode die Zeiten und Anteile der Messperiode zu addieren, in denen der Atem-Unruhe-Index im Bereich für Tiefschlaf, Leichtschlaf, REM-Schlaf oder Wachzustand lag, und die Schlafqualität basierend auf der Dauer des erfassten Tiefschlafs zu ermitteln.

2. Atemgasanalysator (10) nach Anspruch 1, wobei der Atemgasanalysator (10) dazu eingerichtet ist, die Schlafqualität als Schlafqualitätsindex wiederzugeben.

3. Atemgasanalysator (10) nach einem der vorhergehenden Ansprüche, wobei die mindestens eine Vorverarbeitungseinheit (12) dazu eingerichtet ist, den mindestens einen ermittelten oder bereitgestellten Atemgasparameter auszuwerten und ferner mindestens eine Kennzahl zu ermitteln, wobei die mindestens eine Kennzahl aus mindestens einem der folgenden Atemgasparameter ermittelt wird: Atemgasfluss, Atemgaskontur, inspiratorisches Tidalvolumen, exspiratorisches Tidalvolumen, Atemfrequenz, Atemzugsdauer, Inspirationsdauer, Exspirationsdauer, Peakflow, Leckage.

4. Atemgasanalysator (10) nach Anspruch 3, wobei die mindestens eine Zwischenspeichereinheit (13) dazu eingerichtet ist, die mindestens eine Kennzahl zwischenzuspeichern, und die mindestens eine Detektoreinheit (14) dazu eingerichtet ist, das mindestens eine Schlafstadium ferner aus der mindestens einen zwischengespeicherten Kennzahl zu ermitteln.

5. Atemgasanalysator (10) nach einem der vorhergehenden Ansprüche, wobei der Atemgasanalysator (10) dazu eingerichtet ist, basierend auf dem ermittelten Stabilitätslevel und/oder dem ermittelten Schlafstadium und/oder der ermittelten Schlafqualität ein Polygraphie- oder Diagnosegerät und/oder ein PAP- oder Beatmungsgerät zu steuern.

6. Atemgasanalysator (10) nach einem der vorhergehenden Ansprüche, wobei der Atemgasanalysator (10) dazu eingerichtet ist, das Schlafstadium und/oder das Stabilitätslevel und/oder einen Schlafqualitätsindex an ein Bedien- und Informationssystem (22) oder an eine Anzeige (23) eines Beatmungsgerätes (33) zu übermitteln, wobei das Bedien- und Informationssystem (22) oder die Anzeige (23) dazu eingerichtet ist, das Schlafstadium und/oder das Stabilitätslevel und/oder den Schlafqualitätsindex darzustellen.

7. Atemgasanalysator (10) nach einem der vorhergehenden Ansprüche, ferner umfassend eine Schnittstelle (17), die dazu eingerichtet ist, mit einem Endgerät (20) zu interagieren, um die Schlafqualität auf dem Endgerät (20) darzustellen.

8. Beatmungsgerät, umfassend einen Atemgasanalysator (10) nach einem der vorhergehenden Ansprüche.

9. Gerät zur Durchführung einer Polygraphie und/oder einer Polysomnographie, umfassend einen Atemgasanalysator nach einem der Ansprüche 1 bis 7.

10. Verfahren zur Steuerung eines Atemgasanalysators (10) nach einem der Ansprüche 1 bis 7, umfassend die folgenden Schritte:
a. Beziehen mindestens eines Atemgasparameters des Atemgases durch den Atemgasanalysator (10) von einem Messsystem (11) oder Ermitteln mindestens eines Atemgasparameters aus dem Atemgas durch die mindestens eine Messeinheit;
b. Auswerten des mindestens einen ermittelten oder bereitgestellten Atemgasparameters und Ermitteln mindestens eines Atemgassignals durch die mindestens eine Vorverarbeitungseinheit (12), wobei das mindestens eine Atemgassignal ein Atemgasvolumen eines aktuellen Atemzuges ist und ein aktuelles Atemgasvolumen pro Atemzug als Prozentwert eines Normalwertes eines inspiratorischen Atemminutenvolumens einer bestimmten Zeit normiert wird, um einen normierten Wert, insbesondere ein normiertes Atemgasvolumen, zu ermitteln;
c. Zwischenspeichern des normierten Wertes durch die mindestens eine Zwischenspeichereinheit (13);
d. Ermitteln mindestens eines Schlafstadiums aus mindestens einem zwischengespeicherten normierten Wert durch die mindestens eine Detektoreinheit (14), wobei eine Auslenkung des normierten Werts vom Normalwert ermittelt wird, die ermittelte Auslenkung mit einem Tiefpass gefiltert wird, der derart geglättete Wert als ein der Auslenkung entsprechender Atem-Unruhe-Index wiedergegeben wird und ein aktueller Atemzug entsprechend dem ermittelten Wert des Atem-Unruhe-Index oder entsprechend einem Stabilitätslevel als Kehrwert des Atem-Unruhe-Index einem Schlafstadium zugeordnet wird;
e. Ermitteln einer Schlafqualität basierend auf dem mindestens einen durch die Detektoreinheit (14) ermittelten Schlafstadium durch die mindestens eine Integratoreinheit (15), wobei am Ende einer Messperiode die Zeiten und Anteile der Messperiode addiert werden, in denen der Atem-Unruhe-Index im Bereich für Tiefschlaf, Leichtschlaf, REM-Schlaf oder Wachzustand lag, und die Schlafqualität basierend auf der Dauer des erfassten Tiefschlafs ermittelt wird;
f. Darstellen der Schlafqualität als Schlafqualitätsindex.

11. Verfahren nach Anspruch 10, wobei der Atemgasanalysator (10) ein Atemgasanalysator nach Anspruch 7 ist, ferner umfassend: Interagieren mit einem Endgerät (20) über die Schnittstelle (17), um die Schlafqualität auf dem Endgerät (20) darzustellen.

12. Verfahren nach Anspruch 10 oder 11, wobei der Atemgasanalysator (10) ein Atemgasanalysator nach Anspruch 5 ist, ferner umfassend: Steuern eines Polygraphie- oder Diagnosegeräts basierend auf dem ermittelten Stabilitätslevel und/oder dem ermittelten Schlafstadium und/oder der ermittelten Schlafqualität.

## Claims

1. A respiratory gas analyzer (10) for evaluating a respiratory gas, wherein the respiratory gas analyzer (10) is configured to obtain at least one respiratory gas parameter of the respiratory gas from a measuring system (11) or comprises at least one measuring unit which is configured to determine at least one respiratory gas parameter from the respiratory gas, wherein the respiratory gas analyzer (10) comprises:
- at least one pre-processing unit (12) which is configured to evaluate the at least one determined or provided respiratory gas parameter and to determine at least one respiratory gas signal, wherein the at least one respiratory gas signal is a respiratory gas volume of a current breath and the pre-processing unit is configured to normalize a current respiratory gas volume per breath as a percentage of a normal value of an inspiratory respiratory minute volume of a specific time in order to determine a normalized value, in particular a normalized respiratory gas volume;
- at least one temporary storage unit (13) which is configured to temporarily store the normalized value;
- at least one detector unit (14) which is configured to determine at least one stage of sleep from at least one temporarily stored normalized value, wherein the detector unit is configured to determine a deviation of the normalized value from the normal value, to filter the determined deviation with a low pass, to reproduce the value smoothed in this manner as a respiratory disturbance index corresponding to the deviation, and to assign a current breath to a stage of sleep according to the determined value of the respiratory disturbance index or according to a stability level as a reciprocal of the respiratory disturbance index;
- at least one integrator unit (15) which is configured to determine a quality of sleep based on the at least one stage of sleep determined by means of the detector unit (14), wherein the integrator unit is configured, at the end of a measuring period, to add up the times and proportions of the measuring period during which the respiratory disturbance index was in the range for deep sleep, light sleep, REM sleep, or waking state, and to determine the quality of sleep based on the duration of the detected deep sleep.

2. The respiratory gas analyzer (10) according to claim 1, wherein the respiratory gas analyzer (10) is configured to reproduce the quality of sleep as a sleep quality index.

3. The respiratory gas analyzer (10) according to any one of the preceding claims, wherein the at least one pre-processing unit (12) is configured to evaluate the at least one determined or provided respiratory gas parameter and also to determine at least one characteristic value, wherein the at least one characteristic value is determined from at least one of the following respiratory gas parameters: respiratory gas flow, respiratory gas contour, inspiratory tidal volume, expiratory tidal volume, respiratory rate, respiratory duration, inspiratory duration, expiratory duration, peak flow, leakage.

4. The respiratory gas analyzer (10) according to claim 3, wherein the at least one temporary storage unit (13) is configured to temporarily store the at least one characteristic value, and the at least one detector unit (14) is configured to also determine the at least one stage of sleep from the at least one temporarily stored characteristic value.

5. The respiratory gas analyzer (10) according to any one of the preceding claims, wherein the respiratory gas analyzer (10) is configured to control a polygraph device or diagnostic device and/or a PAP machine or ventilator based on the determined stability level and/or the determined stage of sleep and/or the determined quality of sleep.

6. The respiratory gas analyzer (10) according to any one of the preceding claims, wherein the respiratory gas analyzer (10) is configured to transmit the stage of sleep and/or the stability level and/or a sleep quality index to an operating and information system (22) or to a display (23) of a ventilator (33), wherein the operating and information system (22) or the display (23) is configured to represent the stage of sleep and/or the stability level and/or the sleep quality index.

7. The respiratory gas analyzer (10) according to any one of the preceding claims, further comprising an interface (17) which is configured to interact with a terminal (20) in order to represent the quality of sleep on the terminal (20).

8. A ventilator, comprising a respiratory gas analyzer (10) according to any one of the preceding claims.

9. A device for performing a polygraph and/or a polysomnography, comprising a respiratory gas analyzer according to any one of claims 1 to 7.

10. A method for controlling a respiratory gas analyzer (10) according to any one of claims 1 to 7, comprising the following steps:
a. obtaining at least one respiratory gas parameter of the respiratory gas from a measuring system (11) by means of the respiratory gas analyzer (10) or determining at least one respiratory gas parameter from the respiratory gas by means of the at least one measuring unit;
b. evaluating the at least one determined or provided respiratory gas parameter and determining at least one respiratory gas signal by means of the at least one pre-processing unit (12), wherein the at least one respiratory gas signal is a respiratory gas volume of a current breath and a current respiratory gas volume per breath is normalized as a percentage of a normal value of an inspiratory respiratory minute volume of a specific time in order to determine a normalized value, in particular a normalized respiratory gas volume;
c. temporarily storing the normalized value by means of the at least one temporary storage unit (13);
d. determining at least one stage of sleep from at least one temporarily stored normalized value by means of the at least one detector unit (14), wherein a deviation of the normalized value from the normal value is determined, the determined deviation is filtered with a low pass, the value smoothed in this manner is reproduced as a respiratory disturbance index corresponding to the deviation, and a current breath is assigned to a stage of sleep according to the determined value of the respiratory disturbance index or according to a stability level as a reciprocal of the respiratory disturbance index;
e. determining, by means of the at least one integrator unit (15), a quality of sleep based on the at least one stage of sleep determined by means of the detector unit (14), wherein the times and proportions of the measuring period during which the respiratory disturbance index was in the range for deep sleep, light sleep, REM sleep, or waking state are added up at the end of a measuring period, and the sleep quality is determined based on the duration of the detected deep sleep;
f. representing the quality of sleep as a sleep quality index.

11. The method according to claim 10, wherein the respiratory gas analyzer (10) is a respiratory gas analyzer according to claim 7, further comprising: interacting with a terminal (20) via the interface (17) in order to represent the quality of sleep on the terminal (20).

12. The method according to claim 10 or 11, wherein the respiratory gas analyzer (10) is a respiratory gas analyzer according to claim 5, further comprising: controlling a polygraph or diagnostic device based on the determined stability level and/or the determined stage of sleep and/or the determined quality of sleep.

## Revendications

1. Analyseur de gaz respiratoire (10) permettant d'évaluer un gaz respiratoire, dans lequel l'analyseur de gaz respiratoire (10) est configuré pour obtenir au moins un paramètre de gaz respiratoire du gaz respiratoire à partir d'un système de mesure (11), ou comporte au moins une unité de mesure, laquelle est configurée pour déterminer au moins un paramètre de gaz respiratoire à partir du gaz respiratoire, dans lequel l'analyseur de gaz respiratoire (10) comporte :
- au moins une unité de prétraitement (12) configurée pour évaluer l'au moins un paramètre de gaz respiratoire déterminé ou mis à disposition et pour déterminer au moins un signal de gaz respiratoire, dans lequel l'au moins un signal de gaz respiratoire est un volume de gaz respiratoire d'une respiration actuelle et l'unité de prétraitement est configurée pour normaliser un volume de gaz respiratoire actuel par respiration en tant que pourcentage d'une valeur normale d'une ventilation minute inspiratoire d'un temps donné, afin de déterminer une valeur normalisée, en particulier un volume de gaz respiratoire normalisé ;
- au moins une unité de stockage temporaire (13) configurée pour stocker temporairement la valeur normalisée ;
- au moins une unité de détecteur (14) configurée pour déterminer au moins un stade de sommeil à partir d'au moins une valeur normalisée stockée temporairement, dans lequel l'unité de détecteur est configurée pour déterminer une déviation de la valeur normalisée par rapport à la valeur normale, pour filtrer la déviation déterminée à l'aide d'un filtre passe-bas, pour restituer la valeur ainsi lissée en tant qu'indice de trouble respiratoire correspondant à la déviation et pour attribuer une respiration actuelle à un stade de sommeil en fonction de la valeur déterminée de l'indice de trouble respiratoire ou en fonction d'un niveau de stabilité en tant que valeur inverse de l'indice de trouble respiratoire ;
- au moins une unité d'intégrateur (15) configurée pour déterminer une qualité de sommeil sur la base de l'au moins un stade de sommeil déterminé par l'unité de détecteur (14), dans lequel l'unité d'intégrateur est configurée pour ajouter, à la fin d'une période de mesure, les temps et les parts de la période de mesure pendant lesquels l'indice de trouble respiratoire se trouvait dans la plage correspondant au sommeil profond, au sommeil léger, au sommeil paradoxal ou à l'état de veille, et pour déterminer la qualité de sommeil sur la base de la durée du sommeil profond détecté.

2. Analyseur de gaz respiratoire (10) selon la revendication 1, dans lequel l'analyseur de gaz respiratoire (10) est configuré pour restituer la qualité du sommeil en tant qu'indice de qualité de sommeil.

3. Analyseur de gaz respiratoire (10) selon l'une des revendications précédentes, dans lequel l'au moins une unité de prétraitement (12) est configurée pour évaluer l'au moins un paramètre de gaz respiratoire déterminé ou mis à disposition et en outre pour déterminer au moins un nombre caractéristique, dans lequel l'au moins un nombre caractéristique est déterminé à partir de l'un au moins parmi les paramètre de gaz respiratoire suivants : flux de gaz respiratoire, contour de gaz respiratoire, volume tidal inspiratoire, volume tidal expiratoire, fréquence respiratoire, durée de respiration, durée d'inspiration, durée d'expiration, flux de pointe, fuite.

4. Analyseur de gaz respiratoire (10) selon la revendication 3, dans lequel l'au moins une unité de stockage temporaire (13) est configurée pour stocker temporairement l'au moins un nombre caractéristique, et l'au moins une unité de détecteur (14) est configurée pour déterminer l'au moins un stade de sommeil également à partir de l'au moins un nombre caractéristique stocké temporairement.

5. Analyseur de gaz respiratoire (10) selon l'une des revendications précédentes, dans lequel l'analyseur de gaz respiratoire (10) est configuré pour commander un appareil de polygraphie ou de diagnostic et/ou un appareil PAP ou de ventilation, sur la base du niveau de stabilité déterminé et/ou du stade de sommeil déterminé et/ou de la qualité de sommeil déterminée.

6. Analyseur de gaz respiratoire (10) selon l'une des revendications précédentes, dans lequel l'analyseur de gaz respiratoire (10) est configuré pour transmettre le stade de sommeil et/ou le niveau de stabilité et/ou un indice de qualité de sommeil à un système d'exploitation et d'information (22) ou à un affichage (23) d'un appareil de ventilation (33), dans lequel le système d'exploitation et d'information (22) ou l'affichage (23) est configuré pour représenter le stade de sommeil et/ou le niveau de stabilité et/ou l'indice de qualité de sommeil.

7. Analyseur de gaz respiratoire (10) selon l'une des revendications précédentes, comportant en outre une interface (17) configurée pour interagir avec un appareil terminal (20) afin de représenter la qualité de sommeil sur l'appareil terminal (20).

8. Appareil de ventilation, comportant un analyseur de gaz respiratoire (10) selon l'une des revendications précédentes.

9. Appareil d'exécution d'une polygraphie et/ou d'une polysomnographie, comportant un analyseur de gaz respiratoire selon l'une des revendications 1 à 7.

10. Procédé de commande d'un analyseur de gaz respiratoire (10) selon l'une des revendications 1 à 7, comportant les étapes suivantes :
a. obtention d'au moins un paramètre de gaz respiratoire du gaz respiratoire par l'analyseur de gaz respiratoire (10) à partir d'un système de mesure (11) ou détermination d'au moins un paramètre de gaz respiratoire à partir du gaz respiratoire par l'au moins une unité de mesure ;
b. évaluation de l'au moins un paramètre de gaz respiratoire déterminé ou mis à disposition et détermination d'au moins un signal de gaz respiratoire par l'au moins une unité de prétraitement (12), dans lequel l'au moins un signal de gaz respiratoire est un volume de gaz respiratoire d'une respiration actuelle et un volume de gaz respiratoire actuel par respiration est normalisé en tant que pourcentage d'une valeur normale d'une ventilation minute inspiratoire d'un temps donné, afin de déterminer une valeur normalisée, en particulier un volume de gaz respiratoire normalisé ;
c. stockage temporaire de la valeur normalisée par l'au moins une unité de stockage temporaire (13) ;
d. détermination d'au moins un stade de sommeil à partir d'au moins une valeur normalisée stockée temporairement par l'au moins une unité de détecteur (14), dans lequel une déviation de la valeur normalisée par rapport à la valeur normale est déterminée, la déviation déterminée est filtrée à l'aide d'un filtre passe-bas, la valeur ainsi lissée est restituée en tant qu'indice de trouble respiratoire correspondant à la déviation et une respiration actuelle est attribuée à un stade de sommeil en fonction de la valeur déterminée de l'indice de trouble respiratoire ou en fonction d'un niveau de stabilité en tant que valeur inverse de l'indice de trouble respiratoire ;
e. détermination d'une qualité de sommeil, par l'au moins une unité d'intégrateur (15), sur la base de l'au moins un stade de sommeil déterminé par l'unité de détecteur (14), dans lequel, à la fin d'une période de mesure, les temps et les parts de la période de mesure pendant lesquels l'indice de trouble respiratoire se trouvait dans la plage correspondant au sommeil profond, au sommeil léger, au sommeil paradoxal ou à l'état de veille sont ajoutés, et la qualité de sommeil est déterminée sur la base de la durée du sommeil profond détecté ;
f. représentation de la qualité du sommeil en tant qu'indice de qualité de sommeil.

11. Procédé selon la revendication 10, dans lequel l'analyseur de gaz respiratoire (10) est un analyseur de gaz respiratoire selon la revendication 7, comportant en outre : l'interaction avec un appareil terminal (20) par le biais de l'interface (17), afin de représenter la qualité de sommeil sur l'appareil terminal (20).

12. Procédé selon la revendication 10 ou 11, dans lequel l'analyseur de gaz respiratoire (10) est un analyseur de gaz respiratoire selon la revendication 5, comportant en outre : la commande d'un appareil de polygraphie ou de diagnostic sur la base du niveau de stabilité déterminé et/ou du stade de sommeil déterminé et/ou de la qualité de sommeil déterminée.
